# EUROPÄISCHE PATENTSCHRIFT

(11) **EP 1 637 172 B1**
(45) Veröffentlichungstag und Bekanntmachung des Hinweises auf die Patenterteilung: **28.05.2008**
(21) Anmeldenummer: 04022381.0
(22) Anmeldetag: 21.09.2004
(51) Int. Cl.: A61M 1/02, B04B 5/04

(54) **Vorrichtung und Verfahren zum Trennen von in Beuteln befindlichen Blutkomponenten**
Device and method for separating blood components stored in bags
Dispositif et méthode de séparation des composantes du sang contenues dans des sacs

(43) Veröffentlichungstag der Anmeldung: 22.03.2006
(73) Patentinhaber: Andreas Hettich GmbH & Co. KG, 78532 Tuttlingen (DE)
(72) Erfinder: Eberle, Günter, 78532 Tuttlingen (DE)
(74) Vertreter: Riebling, Peter

(56) Entgegenhaltungen:
- EP-A- 0 278 043
- EP-A- 0 416 495
- WO-A-92/00145
- US-A- 4 539 005

## Beschreibung

Die Erfindung betrifft eine Vorrichtung und ein Verfahren zum Trennen von in Beuteln befindlichen Blutkomponenten, nach dem Oberbegriff der unabhängigen Patentansprüche 1 und 8.

Im folgenden wird lediglich ein Trennen von Vollblut in Blutplasma und Erythrozyten (rote Blutkörperchen) beschrieben, was jedoch für die Erfindung nicht einschränkend sein soll. Natürlich kann die Erfindung auch für eine weitere Trennung von bereits abgetrennten Blutkomponenten eingesetzt werden, so z.B. zur Herstellung von Thrombozytenkonzentrat aus gepoolten Buffycoats. Vom Erfindungsgedanken umfasst sein soll daher jede Trennung von einer oder mehreren Blutkomponente(n) aus Vollblut oder aus einem beliebigen Gemisch von mehreren Blutkomponenten.

Als **Stand der Technik** wird von einem herkömmlichen mit Blut/Blutkomponenten gefüllten flexiblen Entnahmebeutel und über einen Verbindungsschlauch damit verbundenen Satellitenbeutel zur Aufnahme einer abgetrennten Blutkomponente und ggf. einem Nährlösungsbeutel ausgegangen, wobei die Beutel gemeinsam in einen herkömmlichen (Doppel-)Blutbeutelbecher eingesetzt und zentrifugiert werden. Danach werden die Beutel aus dem Becher genommen und offen einzeln in ein Abpressgerät gehängt und durch Abpressen die Blutkomponenten voneinander getrennt.

Die WO 92/00145 A offenbart zur Bluttrennung einen speziellen Doppelbecher mit zwei Einzelbechern und je einer Doppelkammer, wobei in die eine Kammer jedes der beiden Einzelbecher mindestens ein Entnahmebeutel einsetzbar ist und in die andere Kammer jedes der beiden Einzelbecher mindestens ein über Verbindungsschläuche mit dem jeweiligen Entnahmebeutel verbundener zugehöriger Satellitenbeutel für das abgetrennte Blutplasma einsetzbar ist.

**Nachteil** dabei ist, dass die Beutel sich beim Zentrifugieren stark aneinander und an die Becherwandung (insbesondere auf den Becherboden) pressen und nach dem Zentrifugieren beim Entnehmen der Beutel aus dem Becher und beim Trennen der Beutel voneinander eine relativ große Kraft und Geschicklichkeit aufgewendet werden muss, die zu einer Beschädigungsgefahr der Beutel und Schläuche und zu relativ starken unerwünschten Erschütterungen des Entnahmebeutels und damit der Trennschicht zwischen den abgetrennten Blutkomponenten führt. Dadurch mischen sich die abgetrennten Blutkomponenten im Bereich der Trennschicht teilweise wieder und das Volumenverhältnis der abgetrennten Blutkomponenten zueinander ändert sich, d.h. der Trenn-Wirkungsgrad sinkt unerwünscht. Weiterer Nachteil ist, dass die Beutel insbesondere im nicht vollständig gefüllten Zustand nicht optimal in den Becher passen und dadurch sich Falten und damit Sedimentationsnester während der Zentrifugation bilden können.

Die US 4539005 A offenbart einen Apparat und ein Verfahren zur schnellen Infusion von Blut oder anderen Flüssigkeiten in einen Patienten, wobei der Apparat eine Halterung aufweist, die Luftanschlüsse mit Rückschlagventilen für Aufpumpbeutel besitzt, die durch mindestens eine Pumpe der Vorrichtung aufpumpbar sind.
**Aufgabe** der vorliegenden Erfindung ist es daher, eine Vorrichtung und ein Verfahren zum Trennen von in Beuteln befindlichen Blutkomponenten, sowie einen zugehörigen Blutbeutelbecher so weiter zu bilden, dass der Trenn-Wirkungsgrad dadurch erhöht wird, dass die durch Zentrifugieren voneinander getrennten im Entnahmebeutel befindlichen Blutkomponenten bis zu ihrer vollständigen Abtrennung voneinander durch eine Abpressvorrichtung möglichst stabil über die Trennschicht getrennt voneinander verbleiben und sich nicht oder nur unwesentlich wieder vermischen.

Zur **Lösung** der Aufgabe dienen die Merkmale der unabhängigen Patentansprüche 1 und 8.

Wesentlich für die **Vorrichtung** zum Trennen von in Beuteln befindlichen Blutkomponenten ist hierbei, dass die Vorrichtung eine Becherhalterung für einen speziellen Doppelbecher mit zwei Einzelbechern und je einer Doppelkammer beinhaltet, wobei in die eine Kammer jedes der beiden Einzelbecher mindestens ein Entnahmebeutel und mindestens ein Aufpumpbeutel einsetzbar ist und in die andere Kammer jedes der beiden Einzelbecher mindestens ein über Verbindungsschläuche mit dem jeweiligen Entnahmebeutel verbundener zugehöriger Satellitenbeutel für das abgetrennte Blutplasma (und ggf. für eine dem Entnahmebeutel zuzuführende Nährlösung) einsetzbar ist, wobei die Vorrichtung mindestens einen Sensor, bevorzugt je einen Sensor, zur Erkennung der Trennschicht zwischen den abgetrennten Blutkomponenten in den Verbindungsschläuchen zwischen den Entnahmebeuteln und den zugehörigen Satellitenbeuteln beinhaltet.

Insbesondere ist die Pumpe zum Aufpumpen der Aufpumpbeutel als Kreiselpumpe ausgebildet, kann aber auch eine Hubkolbenpumpe oder Membranpumpe oder ähnliches sein, deren Drehzahl den herrschenden Druck in den Aufpumpbeuteln bestimmt.

Optional sind getaktet angesteuerte Magnetklemmventile vorgesehen, über welche die Verbindungsschläuche zwischen den Entnahmebeuteln und den Satellitenbeuteln geführt sind, so dass die Durchflussmenge fein gesteuert werden kann, damit die Trennschicht im Entnahmebeutel stabil bleibt und sich keine Verwirbelungen ergeben.

Weiterhin ist optional vorgesehen, dass die an den Entnahmebeuteln anliegenden Aufpumpbeutel auf mindestens zwei unterschiedliche Druckniveaus p1 und p2 aufpumpbar sind, so dass die Aufpumpbeutel zum einen vor der Zentrifugation leicht auf p1 aufgepumpt werden können und die Entnahmebeutel damit gegen die Becherwand pressen, um Faltenbildung und Sedimentnester zu vermeiden, und zum anderen die Aufpumpbeutel auf einen erhöhten Druck p2 > p1 aufgepumpt werden können, um nach der Zentrifugation die innerhalb des Entnahmebeutels befindlichen, voneinander über die Trennschicht getrennten Blutkomponenten abzupressen und daher vollständig zu trennen, wobei die abgetrennte Blutkomponenten in einem der Satellitenbeutel aufgefangen wird.

Insbesondere sind für die die beiden Drucke p1 und p2 innerhalb der Vorrichtung zwei verschiedene Positionen des Bechers vorgesehen, die insbesondere über einen verschiebbaren Längsschlitten erreicht werden, auf dem die Becherhalterung und der Becher mit den Beuteln sich befindet. Bevorzugt ist der niedrigere Druck p1 für das "Vorspannen" der Beutel einer Ausgefahrenen Position des Längsschlittens zugeordnet, der höhere Druck p2 für das Abpressen bzw. Trennen der Blutkomponenten voneinander.

Weiterhin besitzt die Vorrichtung bevorzugt eine vertikal sich erstreckendes Aufhängegestell für die Satellitenbeutel, wobei erste Haken für den Beutel der abgetrennten Blutkomponente und zweite Haken für den Beutel einer Nährlösung vorgesehen sind, wobei der Beutel für die Nährlösung sich vertikal höher befindet, als der Beutel für die abgetrennte Blutkomponente, da die Nährlösung insbesondere nur über Schwerkraft angetrieben und durch eines der getakteten Magnetklemmventile in den Entnahmebeutel eingeführt wird.

Für die Funktionen "Start", "Magnetventil" und "Reset" ist je ein Bedienelement vorgesehen.

Wesentlich für das **Verfahren** zum Trennen von in Beuteln befindlichen Blutkomponenten sind hierbei folgende Schritte:
1. Bestücken des Doppelkammer-Doppelbechers mit den Entnahme- und Satellitenbeuteln,
2. Einsetzen des Doppelkammer-Doppelbechers in die Vorrichtung zum Trennen von in Beuteln befindlichen Blutkomponenten und Aufpumpen der Aufpumpbeutel auf einen unteren Druck p1, bis die Entnahmebeutel satt in ihrer jeweiligen Kammer des Doppelkammer-Doppelbechers liegen,
3. Abnehmen des Doppelkammer-Doppelbechers von der Vorrichtung und Einsetzen in eine geeignete Zentrifuge und Zentrifugieren,
4. Nochmaliges Einsetzen des Doppelkammer-Doppelbechers in die Vorrichtung,
5. Entnahme der Satellitenbeutel aus dem Doppelkammer-Doppelbecher und Positionierung der Satellitenbeutel derart, dass die Verbindungsschläuche zwischen den Entnahmebeuteln und den Satellitenbeutel für die abgetrennten Blutkomponenten über mindestens einen Sensor, inesbesondere je einen Sensor, zur Erkennung der Trennschicht geführt werden,
6. Öffnen der Sicherheitsabsperr-Ventile zwischen dem Entnahmebeutel und dem zugehörigen Satellitenbeutel,
7. Aufpumpen der Aufpumpbeutel auf einen oberen Druck p2, bis die Trennschicht im Entnahmebeutel in die Verbindungsschläuche zwischen den Entnahmebeuteln und den Satellitenbeutel zum Sensor gewandert ist, der den Aufpumpvorgang der Aufpumpbeutel beendet,
8. Trennen der zugehörigen Satellitenbeutel für die abgetrennte Blutkomponente von den Entnahmebeuteln und/durch Verschließen, insbesondere Verschweißen,
9. Optionales Einfüllen von Nährlösung in die Entnahmebeutel und Trennen der zugehörigen Satellitenbeutel für die Nährlösung von den Entnahmebeuteln und/durch Verschließen, insbesondere Verschweißen,
10. Herausnehmen der Entnahmebeutel aus dem Doppelkammer-Doppelbecher, sowie Entnahme der Satellitenbeutel von der Vorrichtung.

Die Aufpumpbeutel werden nur bei Defekt oder zu Reinigungszwecken aus den Kammern des Doppelkammer-Doppelbechers entnommen, können daher im Regelfall im Becher verbleiben.

Das Aufpumpen der Aufpumpbeutel geschieht hierbei insbesondere über mindestens eine Kreiselpumpe, deren Drehzahl den herrschenden Druck im Aufpumpbeutel bestimmt.

Das Zuführen der Nährlösung aus dem Satellitenbeutel für die Nährlösung in den Entnahmebeutel hinein geschieht hierbei insbesondere über Gravitationskraft, kann aber in einer anderen Ausführungsform mittels Pumpe erfolgen.

Der Volumenstrom der abzutrennenden Blutkomponente (z.B. Blutplasma) wird hierbei insbesondere über getaktete Magnetklemmventile geregelt, um die Trennschicht stabil zu halten.

Auch der Volumenstrom der Nährlösung wird optional über getaktete Magnetklemmventile geregelt.

Diese Magnetklemmventile können aber in einer anderen Ausführungsform entfallen.

Wesentlich für den **Becher** zum Trennen von in Beuteln befindlichen Blutkomponenten ist hierbei, dass dieser als Doppelkammer-Doppelbecher mit zwei Bechern ausgeführt ist, und jeder Becher mit zwei voneinander getrennten Kammern ausgeführt ist, einmal für den Entnahme- und Aufpumpbeutel und einmal für die Satellitenbeutel.

Die beiden Becher sind insbesondere spiegelsymmetrisch und unlösbar über eine Handhabe aneinander befestigt.

Die beiden Kammern der beiden Becher des Doppelbechers für die Entnahme- und Aufpumpbeutel liegen dabei bevorzugt mittelbar oder unmittelbar nebeneinander, und die Kammern für die Satellitenbeutel sind voneinander weiter entfernt angeordnet.

Die jeweilige Kammer für die Satellitenbeutel ist dabei aufklappbar und arretierbar ausgeführt, so dass eine Kammerklappe über ein Scharnier am übrigen Becher angelenkt ist. Insbesondere liegt die Scharnierachse horizontal im Becher, kann aber auch vertikal liegen.

Zwischen den angrenzenden Kammern des Doppelbechers ist ein insbesondere drehbar ausgeführtes Schlauchführungsteil angeordnet, das gleichzeitig als Führung für die Verbindungsschläuche zwischen den Beuteln während des Zentrifugierens und während des Abpressens (Trennens der Blutkomponenten in der Vorrichtung) dient, welche Verbindungsschläuche dadurch eine vertikale Ausrichtung erfahren.

Im Bereich der Handhabe, bevorzugt unterhalb an der Handhabe sind die Rückschlagventile für die Druckluftbeaufschlagung der Aufpumpbeutel angeordnet.

**Vorteile** der oben genannten Vorrichtung und des Verfahrens zum Trennen von in Beuteln befindlichen Blutkomponenten, sowie des zugehörigen Blutbeutelbechers, sind ein extrem hoher Trenn-Wirkungsgrad mit z.B. maximaler Plasmaausbeute, mit reinerem Plasma und einer über 50%-igen Zeitersparnis auf Grund der doppelten Ausführung des Doppelkammer-Doppelbechers.

Im Gegensatz zu den seither üblichen Abpressgeräten, die einer herkömmlichen Blutzentrifugation nachgeschaltet sind, können hier mit einem Schlag die doppelte Anzahl, nämlich zwei Blutbeutel zugleich abgepresst werden, was etwa einer Halbierung der Arbeitszeit entspricht.

Weiterhin müssen die Blutbeutel nicht wie bisher beim Stand der Technik aus den Zentrifugenbechern entnommen werden und offen einzeln in das Abpressgerät eingehängt werden. Das bisher notwendige Entnehmen der Becher verlangt ein Zerren, weil die Blutbeutel sehr straff und luftfrei in die Becher gedrückt worden sind. Das ist bei dem Doppelklappbecher nicht mehr der Fall, die Beutel bleiben drin. Selbst durch sorgfältigstes Arbeiten gelingt es nicht zuverlässig, eine Aufwirbelung, d. h. Vermischen der Randzonen der Sedimente, zu vermeiden.

In dem Doppelklappbecher sind unterhalb des Haltegriffs zwei unabhängig angeschlossene leere Luftbeutel angebracht, deren Luftzufuhr über den Haltegriff beim Aufsetzen auf den Schlitten geschlossen und per Knopfdruck bewirkt wird. In der herausgefahrenen Stellung kann der Doppelbecher mit einem vorwählbaren und eingestellten Luftdruck von ca. 0,2 bar beaufschlagt werden. Dies wird angewendet vor der Zentrifugation, um den eingesetzten Blutbeutel stramm zu pressen und eine Faltenbildung mit Sedimentationsnestern zu vermeiden. Der Luftanschluss am Griff besitzt zwei Rückschlagventile, die den Luftdruck beim Abnehmen aufrecht erhalten.

Nach der Zentrifugation wird der Doppelbecher aufgesetzt und mit dem Schlitten eingeschoben auf die Abpressstellung, wodurch eine Presskraft von 0,5 bar per Knopfdruck erfolgen kann. Bei jedem Aufsetzen der Becher auf den Schlitten werden die Rückschlagventile geöffnet.

Die Satellitenbeutel, d. h. in der Regel ein Leerbeutel für das Plasma und ein weiterer mit bis zu 110 ml Nährlösung für das verbleibende Erythrozytenkonzentrat sowie die Verbindungsschläuche, werden bequem in die seitlichen Klappteile gelegt, die zum Zentrifugieren zugeklappt und nach der Zentrifugation aufgeklappt werden können, um eine leichte Handhabung zu gewährleisten. Auch die Satellitenbeutel neigen dazu, sich in Richtung Boden festzusetzen. Eine Entnahme aus den geöffneten Bechern ist also um vieles einfacher und müheloser.

Die Klappteile selbst besitzen unten ein Scharnier mit Langloch und oben auf beiden Seiten zwei Längshaken, mit dem sie zum kraftschlüssigen Verschließen einfach nach unten geschoben werden und zum Öffnen nach oben.

Über den Haltegriff ist ein um 90° schwenkbarer Flügel angebracht, der zum Beladen in der Längsrichtung wie Abbildung steht und um 90° verdreht wird, um den Anschlussschlauch zum Satellitenbeutel in senkrechter Lage zu halten und ein Umknicken zu verhindern.

Die erfindungsgemäße Vorrichtung besitzt bevorzugt ein Drahtgestell mit Einhängehaken, in den die Satellitenbeutel eingehängt werden. Der Aufnahmebeutel für das Plasma in den jeweils unteren Haken, der Nährbeutel für das Erythrozytenkonzentrat in den oberen Haken. Die Zuleitungen werden über die senkrecht angeordneten Sensoren geführt und weiter über die Abpressventile. Das hat den wesentlichen Vorteil, dass der Abpressfluss des Plasmas in weitgehend senkrechter Anordnung bis zum Sensor erfolgt, was eine Vermeidung von Schlierenbildung und eine weitaus bessere Ausbeute von Plasma zur Folge hat.

Die erfindungsgemäße Vorrichtung ist ein Blutkomponentenseparator insbesondere zur automatischen Trennung von Plasma und Erythrozytenkonzentrat aus vorfiltrierten Blutkonserven in T&T Blutbeutelsystemen oder zur Herstellung von Thrombozytenkonzentraten aus Pool Buffy Coats.

Bei Verwendung von herkömmlichen Separatoren müssen die zentrifugierten Konserven aus den Zentrifugenbechern entnommen und an die Abpressvorrichtung angehängt werden. Dabei kann das Sediment wieder aufgewirbelt werden. Die erfindungsgemäße Vorrichtung dagegen presst die Konserven direkt im Zentrifugenbecher ab.

Vorteile: Abpressen erfolgt direkt im Zentrifugeneinsatz. Kein Aufwirbeln der Sedimente durch Herausnehmen der Beutel aus den Zentrifugeneinsätzen nach der Zentrifugation. Reineres Plasma. Einfacheres Handling. Zeitersparnis: 2 Konserven werden gleichzeitig abgepresst. Platzsparend: Kompakte Bauweise. Nimmt wenig Platz weg im Vergleich zu herkömmlichen Separatoren. Maximale Plasmaausbeute durch Positionierung des Sensors am Schlauch.

In der vorliegenden Erfindung handelt es sich darum, einem bekanntermaßen weichen Blutbeutel eine möglichst pralle Fixierung zu verleihen, die von Anfang an eine Faltenbildung vermeidet, um das Entstehen von Sedimentfallen in den Falten zu verhindern. Dazu wird der gefüllte Blutbeutel in den aufklappbaren Einsatzbecher gelegt, der an der Innenseite mit einem leeren Luftbeutel versehen ist. Der klappbare Nebenbecher nimmt die Satellitenbeutel und Schläuche des Blutbeutelsystems auf und wird mit der Trennwand verschlossen, wodurch eine kompakte, geschlossene Becherhälfte bzw. Einsatzhälfte entsteht, die mit einer zweiten Hälfte über einen Haltegriff verbunden ist, wodurch ein Doppelbecher-Blutbeuteleinsatz entsteht, wie er ähnlich seit langem in der Praxis verwendet wird. Mit Hilfe einer Luftpumpe werden die beiden Luftbeutel leicht aufgepumpt, bis die Blutbeutel keine Falten mehr zeigen. Daraufhin werden die Doppeleinsätze in die Metallbecher der Zentrifuge gesetzt zum Zentrifugieren.

Bei der Zentrifugation wird die im Luftbeutel stehende Luft verdrängt und bildet eine Luftblase, die die Anschlussstücke der Schläuche stabilisiert und am Umknicken hindert, was auch hier das Entstehen von unliebsamen Sedimentfallen vermeidet.

Die Anschlussschläuche können zusätzlich über schwimmende Stabilisationsklammern auf die senkrechte Lage beim Verlassen des Blutbeutels getrimmt werden, was sie ebenfalls und zusätzlich am Umknicken und Bilden von Sedimentationsfallen hindert.

Nach erfolgter Zentrifugation werden die doppelten Einsatzbecher aus der Zentrifuge entnommen, die beiden Nebenbecher aufgeklappt und die an den Verbindungsschläuchen hängenden Satellitenbeutel herausgenommen.

Über eine Luftpumpe werden gleichzeitig beide Luftbeutel weiter aufgepumpt und das im Blutbeutel überstehende Plasma in die Satellitenbeutel verdrängt, wie das genau so zur Gewinnung von gepoolten Thrombozyten gemacht werden kann.

Vorteil: Der zentrifugierte Blutbeutel muss nicht behutsam oder gewaltsam aus seinem Behältnis herausgezogen werden, wodurch mehr oder weniger eine Wiedervermischung stattfindet, sondern die Komponenten bleiben bis zur Auftrennung mit weitgehend scharfen Trennlinien erhalten.

Eine unliebsame Faltenbildung und Umknicken der Anschlussstücke, besonders bei unterfüllten Beuteln, wird vermieden.

Im Folgenden wird die Erfindung anhand von lediglich einen Ausführungsweg darstellende Zeichnungen näher erläutert. Hierbei gehen aus den Zeichnungen und ihrer Beschreibung weitere Merkmale und Vorteile der Erfindung hervor.

Es zeigt:
- Figur 1:: Eine perspektivische Ansicht einer erfindungsgemäßen Vorrichtung zum Trennen von in Beuteln befindlichen Blutkomponenten, ohne Darstellung eines zugehörigen Blutbeutelbechers;
- Figur 2:: Eine perspektivische Ansicht eines Blutbeutelbechers in geöffneter Stellung;
- Figur 3:: Eine perspektivische Ansicht eines Blutbeutelbechers nach Figur 2 in geschlossener Stellung;
- Figur 4:: Eine perspektivische Ansicht einer erfindungsgemäßen Vorrichtung nach Figur 1, mit Darstellung eines zugehörigen Blutbeutelbechers nach den Figuren 2 und 3 in einer in einer ersten Position;
- Figur 5:: Vorrichtung nach Figur 4, mit Darstellung eines zugehörigen Blutbeutelbechers nach den Figuren 2 und 3 in einer zweiten Position.

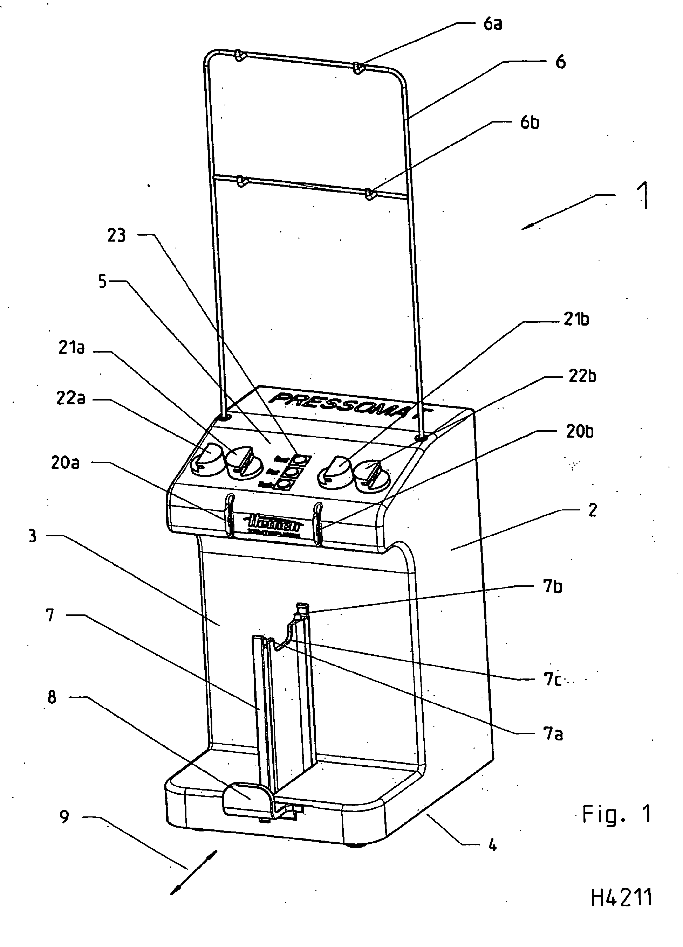

Nach Figur 1 besteht die erfindungsgemäße Vorrichtung 1 zum Trennen von in Beuteln befindlichen Blutkomponenten aus einem Gehäuse 2 und einem daran kopfseitig angebrachten Aufhängegestell 6 zum Aufgängen des Nährlösungsbeutels an den oberen Haken 6a und zum Aufgängen des Plasmabeutels an den unteren Haken 6b, wobei das Aufhängegestell 6 in Form des Buchstabens "A" ausgebildet ist.

Das Gehäuse 2 hat im Frontbereich eine Ausbuchtung 3 für die Aufnahme der Becherhalterung 7 und besteht weiterhin aus einem Boden 4 und einem Kopfbereich 5, sowie Seiten- und Rückwänden.

Im Kopfbereich 5 sind vier Magnetklemmventile 21, 22 vorhanden, zwei davon (21a, 21b) für die Klemmung des Verbindungsschlauches zwischen Entnahme- und Satellitenbeutels und weitere zwei (22a, 22b) für die Klemmung des Verbindungsschlauches zwischen Entnahme- und Nährlösungsbeutels. In der Front des Kopfbereichs 5 ist unterhalb der Magnetklemmventile 21 je eine vertikale Nut eingebracht, in er etwa mittig je ein Sensor 20 (20a, 20b) angeordnet ist, für die Erkennung der Trennschicht in dem jeweiligen Verbindungsschlauch zwischen Entnahme- und Satellitenbeutel. Zwischen den Magnetklemmventilen 21, 22 sind im Kopfbereich 5 drei Betätigungselemente 23 vorgesehen, für "Start", "Magnetventil -ein/aus" und für "Reset".

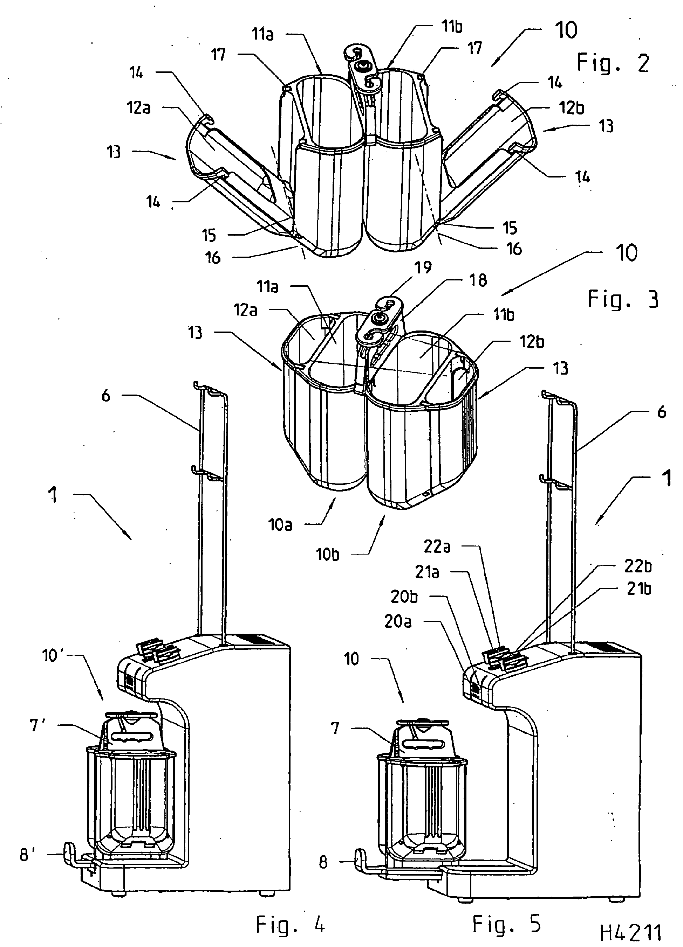

Die Becherhalterung 7 ist für die Aufnahme eines Doppelbechers 10 gemäß den Figuren 2 und 3 ausgestaltet, wobei obere, knopfförmige Anschlüsse 7a, 7b für die Luftzufuhr der Aufblasbeutel angeformt sind. Wird der Doppelbecher 10 gemäß den Figuren 2 und 3 derart auf die Becherhalterung 7 gesetzt, dass ein Einzelbecher 10a auf der einen Seite und der andere Einzelbecher 10b auf der anderen Seite der Becherhalterung 7 zu liegen kommt, dann kuppeln die Anschlüsse 7a, 7b für die Luftzufuhr der Aufblasbeutel an Rückschlagventilen der Einzelbecher 10a, 10b an, wobei diese Rückschlagventile mit den Aufblasbeuteln (nicht dargestellt) in den äußeren Kammern 11a, 11b (siehe Figuren 2, 3) der Einzelbecher 10a, 10b verbunden werden.

Die gesamte Becherhalterung 7 ist auf einem Schlitten 8 gelagert, der in den Schieberichtungen 9 manuell oder motorisch aus/in die Ausbuchtung 3 verschiebbar ausgebildet ist, so dass sich die Positionen nach den Figuren 4 und 5 ergeben. Figur 4 zeigt den Schlitten 8 in seiner ausgefahrenen Position, welche dafür vorgesehen ist, dass die Aufblasbeutel mit einem niedrigen Anfangsdruck (vor der Zentrifugation) aufgepumpt werden, um den Entnahme- und den Aufblasbeutel in der Kammer 11a, 11b kompakt einzupressen. Die Figur 5 zeigt den Schlitten 8 in seiner eingefahrenen Position, welche dafür vorgesehen ist, dass die Aufblasbeutel mit einem höheren Enddruck (nach der Zentrifugation) aufgepumpt werden, um die zentrifugierten Blutkomponenten voneinander endgültig zu trennen, in dem ein Teil aus dem Entnahmebeutel abgedrückt wird mittels des Aufblasbeutels.

In den Figuren 2 und 3 ist nun ein Doppelklappbecher 10 dargestellt, in geöffneter Position (Figur 2) und geschlossener Position (Figur 3).

Der Doppelklappbecher 10 besteht aus zwei Bechern 10a, 10b, die spiegelsymmetrisch mittelbar oder unmittelbar, lösbar oder unlösbar, über eine Handhabe 18 mit ihren Kammern 11a, 11b für die Entnahme- und Aufblasbeutel aneinander angebracht sind. An die jeweilige Kammer 11a, 11b für die Entnahme- und Aufblasbeutel schließt sich je eine Kammer 12a, 12b für die Satellitenbeutel an, welche ein aufklappbares Klappteil 13 jeweils besitzen. Dieses Klappteil 13 ist schwenkbar um eine Achse 16 über ein Scharnier 15 mit dem Rest der Kammer 12a, 12b verbunden, die wiederum mit der Kammer 11a, 11b verbunden ist. Das Klappteil 13 wird beim Einklappen durch Haken 14, die in zugeordnete Aufnahmen 17 rastend eingreifen, gesichert.

Alle Kammern 11, 12 sind nach oben hin geöffnet und nach unten hin geschlossen ausgebildet, so dass die jeweiligen Beutel einfach eingelegt werden können.

Oberhalb der Handhabe 18 ist ein um eine Hochachse drehbares Schlauchführungsteil 19 angeordnet, die in einer horizontalen Ebene drehbar ausgebildet ist. Durch hakenförmige Enden des Schlauchführungsteils 19 mit bogenförmigen Öffnungen werden die Verbindungsschläuche (nicht dargestellt) zwischen den Entnahme- und den Satellitenbeuteln (nicht dargestellt) in einer vorteilhaften vertikalen Lage gehalten, so dass ein optimaler, ungehinderter Flüssigkeitsdurchlass während des Abpressens der abzutrennenden Blutkomponente ermöglicht wird, aber auch zur Verhinderung des Einschlusses von Nestern von ungetrennten Blutkomponenten (z.B. Erythrozytennestern).

### Figurenlegende

1. Vorrichtung zum Trennen von in Beuteln befindlichen Blutkomponenten
2. Gehäuse
3. Ausbuchtung
4. Boden
5. Kopfbereich
6. Aufhängegestell; 6a Haken für Nährlösung, 6b Haken für Plasma
7. Becherhalterung; 7a, b Luftanschluss, 7c Ausnehmung
8. Schlitten für 7
9. Aus-/Einfahrrichtung von 8
10. Doppelbecher; 10a erster Einzelbecher; 10b zweiter Einzelbecher
11. von 12 getrennte Kammer für Entnahmebeutel und Aufblasbeutel; 11a für ersten Einzelbecher; 11b für zweiten Einzelbecher
12. aufklappbare Kammer zur Aufnahme der Satellitenbeutel
13. Klappteil von 12
14. Haken von 12
15. Scharnier zwischen 11 und 12
16. Achse von 15
17. Aufnahmen von 14
18. Handhabe
19. Schlauchführungsteil
20. Sensor für Trennschicht, links: 20a, rechts: 20b
21. Magnetklemmventil für Entnnahmebeutel, links: 21a, rechts: 21b
22. Magnetklemmventil für Nährlösungsbeutel, links: 22a, rechts: 22b
23. Betätigungselemente

## Patentansprüche

1. Vorrichtung zum Trennen von in Beuteln befindlichen Blutkomponenten mit einer Halterung (7) die Luftanschlüsse mit Rückschlagventilen für Aufpumpbeutel besitzt, die durch mindestens eine Pumpe der Vorrichtung aufpumpbar sind, **dadurch gekennzeichnet, dass** die Halterung eine Becherhalterung (7) für einen speziellen Doppelbecher (10) mit zwei Einzelbechern (10a, 10b) und je einer Doppelkammer (11, 12) ist, wobei in die eine Kammer (11) jedes der beiden Einzelbecher (10a, 10b) mindestens ein Entnahmebeutel und mindestens ein Aufpumpbeutel einsetzbar sind und in die andere Kammer (12) jedes der beiden Einzelbecher (10a, 10b) mindestens ein über Verbindungsschläuche mit dem jeweiligen Entnahmebeutel verbundener zugehöriger Satellitenbeutel für das abgetrennte Blutplasma (und ggf. für eine dem Entnahmebeutel zuzuführende Nährlösung) einsetzbar ist, wobei die Vorrichtung mindestens einen Sensor (20), bevorzugt je einen Sensor (20a, 20b) zur Erkennung der Trennschicht zwischen den abgetrennten Blutkomponenten in den Verbindungsschläuchen zwischen den Entnahmebeuteln und den zugehörigen Satellitenbeuteln beinhaltet.

2. **Vorrichtung** nach Anspruch 1, **dadurch gekennzeichnet, dass** die Pumpe zum Aufpumpen der Aufpumpbeutel als Kreiselpumpe, Hubkolbenpumpe oder Membranpumpe ausgebildet ist, deren Drehzahl den herrschenden Druck in den Aufpumpbeuteln bestimmt.

3. **Vorrichtung** nach Anspruch 1 oder 2, **dadurch gekennzeichnet, dass** getaktet angesteuerte Magnetklemmventile (21, 22) vorgesehen sind, über welche die Verbindungsschläuche zwischen den Entnahmebeuteln und den Satellitenbeuteln geführt sind, so dass die Durchflussmenge fein gesteuert werden kann.

4. **Vorrichtung** nach Anspruch 1 bis 3, **dadurch gekennzeichnet, dass** die an den Entnahmebeuteln anliegenden Aufpumpbeutel auf mindestens zwei unterschiedliche Druckniveaus p1 und p2 aufpumpbar sind, so dass die Aufpumpbeutel zum einen vor der Zentrifugation etwas auf p1 aufgepumpt werden können und die Entnahmebeutel damit gegen die Becherwand (10) pressen, um Faltenbildung und Sedimentnester zu vermeiden, und zum anderen die Aufpumpbeutel auf einen erhöhten Druck p2 größer p1 aufgepumpt werden können, um nach der Zentrifugation die innerhalb des Entnahmebeutels befindlichen, voneinander über die Trennschicht getrennten Blutkomponenten abzupressen und daher vollständig zu trennen, wobei die abgetrennten Blutkomponenten in einem der Satellitenbeutel aufgefangen werden.

5. **Vorrichtung** nach Anspruch 1 bis 4, **dadurch gekennzeichnet, dass** für die die beiden Drucke p1 und p2 innerhalb der Vorrichtung zwei verschiedene Positionen des Bechers (10) vorgesehen sind, die insbesondere über einen verschiebbaren Längsschlitten (8) erreicht werden, auf dem die Becherhalterung (7) und der Becher (10) mit den Beuteln sich befindet.

6. **Vorrichtung** nach Anspruch 5, **dadurch gekennzeichnet, dass** der niedrigere Druck p1 für das "Vorspannen" der Beutel einer ausgefahrenen Position des Längsschlittens (8'), der Becherhalterung (7') und der Becher (10') zugeordnet ist, und der höhere Druck p2 für das Abpressen bzw. Trennen der Blutkomponenten voneinander der eingefahrenen Position des Längsschlittens (8), der Becherhalterung (7) und der Becher (10) zugeordnet ist.

7. **Vorrichtung** nach Anspruch 1 bis 6, **dadurch gekennzeichnet, dass** die Vorrichtung ein vertikal sich erstreckendes Aufhängegestell (6) für die Satellitenbeutel besitzt, wobei erste Haken (6b) für den Beutel der abgetrennten Blutkomponenten und zweite Haken (6a) für den Beutel einer Nährlösung vorgesehen sind, wobei der Beutel für die Nährlösung sich vertikal höher befindet, als der Beutel für die abgetrennten Blutkomponenten, da die Nährlösung insbesondere nur über Schwerkraft angetrieben und durch eines der getakteten Magnetklemmventile (22) in den Entnahmebeutel eingeführt wird.

8. **Verfahren** zum Trennen von in Beuteln befindlichen Blutkomponenten mit der Vorrichtung nach einem der Ansprüche 1-7, beinhaltend folgende Schritte:
• Bestücken des Doppelkammer-Doppelbechers (10) mit den Entnahme- und Satellitenbeuteln,
• Einsetzen des Doppelkammer-Doppelbechers (10) in die Vorrichtung (1) zum Trennen von in Beuteln befindlichen Blutkomponenten und Aufpumpen der Aufpumpbeutel auf einen unteren Druck p1, bis die Entnahmebeutel satt in ihrer jeweiligen Kammer (11) des Doppelkammer-Doppelbechers (10) liegen,
• Abnehmen des Doppelkammer-Doppelbechers (10) von der Vorrichtung (1) und Einsetzen in eine geeignete Zentrifuge und Zentrifugieren,
• Nochmaliges Einsetzen des Doppelkammer-Doppelbechers (10) in die Vorrichtung (1),
• Entnahme der Satellitenbeutel aus dem Doppelkammer-Doppelbecher (10) und Positionierung der Satellitenbeutel derart, dass die Verbindungsschläuche zwischen den Entnahmebeuteln und den Satellitenbeuteln für die abgetrennten Blutkomponenten über mindestens einen Sensor (20), insbesondere je einen Sensor (20a, 20b), zur Erkennung der Trennschicht geführt werden,
• Öffnen der Sicherheitsabsperr-Ventile zwischen dem Entnahmebeutel und dem zugehörigen Satellitenbeutel,
• Aufpumpen der Aufpumpbeutel auf einen oberen Druck p2, bis die Trennschicht im Entnahmebeutel in die Verbindungsschläuche zwischen den Entnahmebeuteln und den Satellitenbeutel zum Sensor (20) gewandert ist, der den Aufpumpvorgang der Aufpumpbeutel beendet,
• Trennen der zugehörigen Satellitenbeutel für die abgetrennte Blutkomponente von den Entnahmebeuteln und/durch Verschließen, insbesondere Verschweißen,
• Optionales Einfüllen von Nährlösung in die Entnahmebeutel und Trennen der zugehörigen Satellitenbeutel für die Nährlösung von den Entnahmebeuteln und/durch Verschließen, insbesondere Verschweißen,
• Herausnehmen der Entnahmebeutel aus dem Doppelkammer-Doppelbecher (10), sowie Entnahme der Satellitenbeutel von der Vorrichtung (1).

9. Verfahren nach Anspruch 8, **dadurch gekennzeichnet, dass** die Aufpumpbeutel nur bei Defekt oder zu Reinigungszwecken aus den Kammern des Doppelkammer-Doppelbechers (10) entnommen werden, und im Regelfall im Becher (10) verbleiben.

10. Verfahren nach Anspruch 8 oder 9, **dadurch gekennzeichnet, dass** das Aufpumpen der Aufpumpbeutel insbesondere über mindestens eine Kreiselpumpe oder Hubkolbenpumpe oder Membranpumpe geschieht, deren Drehzahl den herrschenden Druck im Aufpumpbeutel bestimmt.

11. Verfahren nach Anspruch 8 bis 10, **dadurch gekennzeichnet, dass** das Zuführen der Nährlösung aus dem Satellitenbeutel für die Nährlösung in den Entnahmebeutel hinein lediglich über Gravitationskraft geschieht.

12. Verfahren nach Anspruch 8 bis 11, **dadurch gekennzeichnet, dass** der Volumenstrom der abzutrennenden Blutkomponente (z.B. Blutplasma) über getaktete Magnetklemmventile (21) geregelt wird, um die Trennschicht stabil zu halten.

13. Verfahren nach Anspruch 8 bis 12, **dadurch gekennzeichnet, dass** der Volumenstrom der Nährlösung über getaktete Magnetklemmventile (22) geregelt wird.

## Claims

1. Device for separating blood components contained in bags, with a holder (7) which has air connections with check valves for inflatable bags, which can be inflated by at least one pump of the device, **characterised in that** the holder is a cup holder (7) for a special double cup (10) with two single cups (10a, 10b) and a double chamber (11, 12) in each case, wherein at least one collection bag and at least one inflatable bag can be inserted into one chamber (11) of each of the two single cups (10a, 10b) and at least one associated satellite bag, which is connected via connecting hoses to the respective collection bag, and which is for the separated blood plasma (and optionally for a nutrient solution to be supplied to the collection bag) can be inserted into the other chamber (12) of each of the two single cups (10a, 10b), the device having at least one sensor (20), preferably a sensor (20a, 20b) in each case for detection of the separation layer between the separated blood components in the connecting hoses between the collection bags and the associated satellite bags.

2. Device according to claim 1, **characterised in that** the pump for inflating the inflatable bag is configured as a centrifugal pump, reciprocating piston pump or a diaphragm pump, the rotational speed of which determines the prevailing pressure in the inflatable bags.

3. Device according to claim 1 or 2, **characterised in that** magnetic clamp valves (21, 22) which are activated in a clocked manner are provided, via which the connecting hoses are guided between the collection bags and the satellite bags so that the through-flow quantity can be finely controlled.

4. Device according to claim 1 to 3, **characterised in that** the inflatable bags adjacent to the collection bags can be inflated to at least two different pressure levels p1 and p2, so that the inflatable bags can be somewhat inflated, on the one hand, prior to centrifugation, to p1 and therefore press the collection bags against the cup wall (10) to avoid the formation of folds and sedimentation clusters, and, on the other hand, the inflatable bags can be inflated to an increased pressure of p2 greater than p1 in order, after centrifugation, to press out the blood components contained within the collection bag and separated from one another via the separation layer and therefore to completely separate them, the separated blood components being collected in one of the satellite bags.

5. Device according to claim 1 to 4, **characterised in that** for the two pressures p1 and p2 inside the device, two different positions of the cup (10) are provided, which are reached, in particular, via a displaceable longitudinal slide (8), on which the cup holder (7) and the cup (10) with the bags are located.

6. Device according to claim 5, **characterised in that** the low pressure p1 for the "prestressing" of the bags is associated with an extended position of the longitudinal slide (8'), the cup holder (7') and the cups (10'), and the higher pressure p2 for pressing out or separating the blood components from one another is associated with the retracted position of the longitudinal slide (8) of the cup holder (7) and the cups (10).

7. Device according to claim 1 to 6, **characterised in that** the device has a vertically extending suspension frame (6) for the satellite bags, wherein first hooks (6b) for the bag of the separated blood components and second hooks (6a) for the bag of a nutrient solution are provided, the bag for the nutrient solution being located vertically higher than the bag for the separated blood components as the nutrient solution is, in particular, only driven by gravitational force and is introduced into the collection bag through one of the clocked magnetic clamp valves (22).

8. Method for separating blood components contained in bags with the device according to any one of claims 1 to 7 containing the following steps:
• equipping the double chamber double cup (10) with the collection and satellite bags,
• inserting the double chamber double cup (10) into the device (1) for separating blood components contained in bags and inflating the inflatable bags to a lower pressure p1 until the collection bags rest snugly in their respective chamber (11) of the double chamber double cup (10),
• removal of the double chamber double cup (10) from the device (1) and insertion into a suitable centrifuge and centrifugation,
• reinsertion of the double chamber double cup (10) into the device (1),
• removal of the satellite bags from the double chamber double cup (10) and positioning of the satellite bags in such a way that the connecting hoses are guided between the collection bags and the satellite bags for the separated blood components via at least one sensor (20), in particular one sensor (20a, 20b) in each case, for detection of the separation layer,
• opening the safety shut-off valves between the collection bag and the associated satellite bag,
• inflating the inflatable bags to an upper pressure p2 until the separation layer in the collection bag has migrated into the connecting hoses between the collection bags and the satellite bags to the sensor (20), which ends the inflation process of the inflatable bags,
• separation of the associated satellite bags for the separated blood components from the collection bags and/by closing, in particular bonding,
• optional filling of nutrient solution into the collection bags and separation of the associated satellite bags for the nutrient solution from the collection bags and/by closing, in particular bonding,
• removal of the collection bags from the double chamber double cup (10), and removal of the satellite bags from the device (1).

9. Method according to claim 8, **characterised in that** the inflatable bags are only removed from the chambers of the double chamber double cup (10) in the case of a defect or for cleaning purposes, and as a rule remain within the cup (10).

10. Method according to claim 8 or 9, **characterised in that** the inflation of the inflatable bags takes place in particular by means of at least one centrifugal pump or reciprocating piston pump or diaphragm pump, the rotational speed of which determines the prevailing pressure in the inflatable bag.

11. Method according to claim 8 to 10, **characterised in that** the supplying of nutrient solution from the satellite bag for the nutrient solution into the collection bag takes place solely by means of gravitational force.

12. Method according to claim 8 to 11, **characterised in that** the volume flow of the blood components to be separated (for example blood plasma) is controlled via clocked magnetic clamp valves (21) to keep the separation layer stable.

13. Method according to claim 8 to 12, **characterised in that** the volume flow of the nutrient solution is controlled via clocked magnetic clamp valves (22).

## Revendications

1. Dispositif pour séparer des composants sanguins qui se trouvent dans des poches, avec un support (7) qui comporte des raccordements d'air avec des clapets antiretour pour des poches gonflables aptes à être gonflées à l'aide d'au moins une pompe du dispositif, **caractérisé en ce que** le support est constitué par un support de boîtier (7) pour un boîtier double spécial (10) comprenant deux boîtiers individuels (10a, 10b) et des chambres doubles respectives (11, 12), étant précisé qu'on peut placer dans une chambre (11) de chacun des deux boîtiers individuels (10a, 10b) au moins une poche de prélèvement et au moins une poche gonflable, et dans l'autre chambre (12) de chacun des deux boîtiers individuels (10a, 10b) au moins une poche satellite associée qui est reliée à la poche de prélèvement correspondante par des tuyaux de raccordement et qui est prévue pour le plasma sanguin séparé (et éventuellement pour une solution nutritive à amener dans la poche de prélèvement), et que le dispositif contient au moins un capteur (20), de préférence des capteurs respectifs (20a, 20b), pour détecter la couche de séparation entre les composants sanguins séparés, dans les tuyaux de raccordement entre les poches de prélèvement et les poches satellites associées.

2. Dispositif selon la revendication 1, **caractérisé en ce que** la pompe prévue pour gonfler les poches gonflables est conçue comme une pompe centrifuge, une pompe à piston alternatif ou une pompe à membrane dont la vitesse de rotation définit la pression qui règne dans les poches gonflables.

3. Dispositif selon la revendication 1 ou 2, **caractérisé en ce qu'**il est prévu des soupapes de serrage magnétiques (21, 22) à commande synchronisée par lesquelles passent les tuyaux de raccordement entre les poches de prélèvement et les poches satellites, de sorte que le débit peut être commandé avec précision.

4. Dispositif selon les revendications 1 à 3, **caractérisé en ce que** les poches gonflables prévues près des poches de prélèvement sont aptes à être gonflées jusqu'à au moins deux niveaux de pression différents p1 et p2, de sorte qu'elles peuvent d'une part être gonflées légèrement à la pression p1 avant la centrifugation et pressent ainsi les poches de prélèvement contre la paroi du boîtier (10) afin d'éviter la formation de plis et les accumulations de sédiments, et d'autre part être gonflées à une pression accrue p2 supérieure à p1 afin d'extraire par pression, après la centrifugation, les composants sanguins qui se trouvent à l'intérieur de la poche de prélèvement et qui sont séparés les uns des autres par l'intermédiaire de la couche de séparation, et donc de les séparer complètement, les composants sanguins séparés étant recueillis dans l'une des poches satellites.

5. Dispositif selon les revendications 1 à 4, **caractérisé en ce qu'**il est prévu pour les deux pressions p1 et p2 à l'intérieur du dispositif deux positions différentes du boîtier (10), qui sont obtenues en particulier grâce à un chariot longitudinal mobile (8) sur lequel se trouvent le support de boîtier (7) et le boîtier (10) avec les poches.

6. Dispositif selon la revendication 5, **caractérisé en ce que** la pression plus basse p1 prévue pour "tendre préalablement" les poches est associée à une position déployée du chariot longitudinal (8'), du support de boîtier (7') et des boîtiers (10') tandis que la pression plus élevée p2 prévue pour extraire par pression et séparer les composants sanguins les uns des autres est associée à la position rétractée du chariot longitudinal (8), du support de boîtier (7) et des boîtiers (10).

7. Dispositif selon les revendications 1 à 6, **caractérisé en ce qu'**il a une structure de suspension (6) qui s'étend à la verticale pour les poches satellites, des premiers crochets (6b) étant prévus pour la poche des composants sanguins séparés, et des seconds crochets (6a) pour la poche d'une solution nutritive, et la poche pour la solution nutritive se trouvant plus haut, à la verticale, que la poche pour les composants sanguins séparés, étant donné que ladite solution nutritive n'est entraînée que par la force de gravité, notamment, et est introduite dans la poche de prélèvement grâce à l'une des soupapes de serrage magnétiques synchronisées (22).

8. Procédé pour séparer des composants sanguins qui se trouvent dans des poches à l'aide du dispositif selon les revendications 1 à 7, comprenant les étapes suivantes :
- on garnit le boîtier double à chambre double (10) avec les poches de prélèvement et les poches satellites,
- on place le boîtier double à chambre double (10) dans le dispositif (1) pour séparer les composants sanguins qui se trouvent dans les poches, et on gonfle les poches gonflables à une pression inférieure p1 jusqu'à ce que les poches de prélèvement soient parfaitement appliquées contre la chambre correspondante (11) dudit boîtier (10),
- on enlève le boîtier double à chambre double (10) du dispositif (1) et on le place dans une centrifugeuse appropriée, et on procède à la centrifugation,
- on remet le boîtier double à chambre double (10) dans le dispositif (1),
- on enlève les poches satellites du boîtier (10) et on les positionne de telle sorte que les tuyaux de raccordement passent entre les poches de prélèvement et les poches satellites prévues pour les composants sanguins séparés, en passant devant au moins un capteur (20), en particulier des capteurs respectifs (20a, 20b) destinés à détecter la couche de séparation,
- on ouvre les soupapes d'arrêt de sûreté entre la poche de prélèvement et la poche satellite associée,
- on gonfle les poches gonflables à une pression supérieure p2 jusqu'à ce que la couche de séparation dans la poche de prélèvement soit arrivée dans les tuyaux de raccordement entre les poches de prélèvement et les poches satellites jusqu'au capteur (20), qui termine le gonflage des poches gonflables.
- on sépare les poches satellites associées, prévues pour les composants sanguins séparés, des poches de prélèvement et on les ferme/en les fermant, en particulier en les soudant,
- on introduit à titre optionnel une solution nutritive dans les poches de prélèvement et on sépare les poches satellites associées pour la solution nutritive des poches de prélèvement et on les ferme/en les fermant, en particulier en les soudant,
- on retire les poches de prélèvement du boîtier double à chambre double (10) et on enlève les poches de prélèvement du dispositif (1).

9. Procédé selon la revendication 8, **caractérisé en ce que** les poches gonflables ne sont enlevées des chambres du boîtier double à chambre double (10) qu'en cas de défaut ou à des fins de nettoyage, et elles restent généralement dans le boîtier (10).

10. Procédé selon la revendication 8 ou 9, **caractérisé en ce que** le gonflage des poches gonflables se fait en particulier par l'intermédiaire d'au moins une pompe centrifuge, d'une pompe à piston alternatif ou d'une pompe à membrane dont la vitesse de rotation définit la pression qui règne dans la poche gonflable.

11. Procédé selon les revendications 8 à 10, **caractérisé en ce que** le passage de la solution nutritive de la poche satellite destinée à celle-ci à la poche de prélèvement se fait uniquement par gravité.

12. Procédé selon les revendications 8 à 11, **caractérisé en ce que** le débit volumique des composants sanguins à séparer (par exemple le plasma sanguin) est régulé par l'intermédiaire de soupapes de serrage magnétiques synchronisées (21), pour que la couche de séparation reste stable.

13. Procédé selon les revendications 8 à 12, **caractérisé en ce que** le débit volumique de la solution nutritive est régulé par l'intermédiaire de soupapes de serrage magnétiques synchronisées (22).
